# EUROPÄISCHE PATENTSCHRIFT

(11) **EP 1 447 100 B1**
(45) Veröffentlichungstag und Bekanntmachung des Hinweises auf die Patenterteilung: **12.10.2011**
(21) Anmeldenummer: 04002991.0
(22) Anmeldetag: 11.02.2004
(51) Int. Cl.: A61L 2/00, A61L 2/16, A61L 2/23, A01N 33/24, C07C 291/04

(54) **Mikrobizide Wirkstoffkombinationen und daraus hergestellte Desinfektionsmittel**
Microbiocidal compositions and disinfecting agents produced from them
Compositions microbiocidales et des désinfectants produits à partir d'eux

(30) Priorität: 17.02.2003 DE 10306450
(43) Veröffentlichungstag der Anmeldung: 18.08.2004
(73) Patentinhaber: Bode Chemie GmbH, 22525 Hamburg (DE)
(72) Erfinder: Ostermeyer, Christiane, 22559 Hamburg (DE); Pietsch, Hanns Dr., 20148 Hamburg (DE); Meyer, Brigitte, 22309 Hamburg (DE); Bloss, Richard Dr., 25462 Rellingen (DE)
(74) Vertreter: Oltmann, Eckhard

(56) Entgegenhaltungen:
- WO-A-02/12431
- DE-A- 4 331 942
- DE-A- 19 512 588
- FR-A- 2 766 724
- US-B1- 6 168 808

## Beschreibung

Die vorliegende Erfindung betrifft die Verwendung von Wirkstoffkombinationen aus Peroxiden und tert-Alkylamin-N-oxiden als Desinfektionsmittel.

Anorganische Peroxo- und organische Peroxiverbindungen - im folgenden auch unter dem Begriff "Peroxide" zusammengefaßt - sind klassische antimikrobiell wirksame Wirkstoffe mit einem breiten antimikrobiellen Wirkungsspektrum: Sie wirken bakterizid, fungizid, viruzid und sporozid. Im Buch "Praxis der Sterilisation Desinfektion Konservierung Betriebshygiene" von Karl Heinz Wallhäußer, Georg Thieme Verlag, Stuttgart, New York werden Peroxide in der 4. Auflage auf den Seiten 603 bis 607 abgehandelt, in der 5. Auflage auf den Seiten 643 bis 646. Dort werden genannt Wasserstoffperoxid, Dibenzoylperoxid und Natriumperborat.

Weitere im Desinfektionsmittelbereich üblicherweise verwendeten Peroxide sind Peressigsäure (5. Auflage S. 511), Monopersulfate und Peroxodisulfate sowie Magnesium-mono-perphthalat. Diese Wirkstoffe werden zu unterschiedlichen Zwecken verwendet: Besonders das Magnesium-mono-perphthalat ist wegen seiner hohen Wirksamkeit in Verbindung mit einer hervorragenden Materialverträglichkeit, Hautverträglichkeit und sogar Schleimhaut- und Wundverträglichkeit geeignet für alle Arten der Desinfektion: Flächendesinfektion, Instrumentendesinfektion, Hände-, Haut-, Schleimhaut- und Wunddesinfektion. Magnesium-mono-perphthalat ist daher auch der Peressigsäure überlegen, die zwar hinsichtlich der Wirksamkeit wesentlich stärker ist, jedoch hinsichtlich ihrer Korrosivität und ihres Geruches deutliche Nachteile aufweist.

In der europäischen Patentschrift EP 0 818 950 werden Desinfektionsmittelkonzentrate mit einem Gehalt an organischen Persäuren und/oder deren Salzen zur Desinfektion von Flächen und/oder Instrumenten beansprucht.

In der deutschen Offenlegungsschrift DE 195 08 827 werden Wund- und Schleimhautdesinfektionsmittel mit einem Gehalt an Magnesium-mono-perphthalat beansprucht.

In der DOS 197 24 102 werden Mittel zur Schnell-Dekontamination von Haut und Händen mit Magnesiummonoperphthalat beansprucht.

In der DOS 198 08 962 werden Kombinationen von Peroxiden mit Alkylthiouroniumsalzen und/oder α,ω-Alkylendithiouroniumsalzen beansprucht. Diese Kombinationen haben den Nachteil, dass die Thiouroniumsalze nur im sauren oder neutralen Milieu beständig sind und schwer löslich in Wasser sind. Daraus hergestellte Mikroemulsionen hinterlassen auf medizinischen Instrumenten schwerlösliche Rückstände.

In der EP 0873 687 werden Zubereitungen aus Peressigsäure und tert.-N-Alkylaminoxide beansprucht. Wesentliche Nachteile von Peressigsäure sind ihr unangenehmer Geruch und die große Korrosivität, die nur unvollständig durch Korrosionsschutzmittel unterdrückt werden kann.

Bei aller Breite der Wirksamkeit zeigen Peroxide dennoch eine Wirkungsschwäche hinsichtlich der Wirkung gegen Pilze und Hefen. Zwar ist eine Wirksamkeit vorhanden, jedoch werden zur einer gleich starken Reduktion von Pilzen und Hefen wesentlich höhere Konzentrationen benötigt als bei Bakterien. Um das gesamte Keimspektrum einschließlich der Pilze und Hefen abzudecken zu können, müssen Desinfektionsmittel auf Peroxidbasis daher hohe Konzentrationen an Wirkstoff enthalten, die für Pilze und Hefen ausreichend, für die übrigen Keime jedoch überdosiert sind.

In der EP 0 873 687 werden Kombinationen von Peressigsäure, Essigsäure, Wasserstoffperoxid, Aminoxid und Tensid beansprucht. Das Aminoxid dient als sogenannter Remanenzwirkstoff, der die Wirksamkeit des Präparates nach dem Verdunsten der Peressigsäure sicherstellen soll.

DE4331942A1 offenbart Zusammensetzungen zur Reinigung und Desinfektion, welche Peroxid und Alkyldimethylaminoxide enthalten.

Alle Versuche, Magnesium-mono-perphthalat mit fungiziden Wirkstoffen - wie z. B. Thiouroniumsalzen oder quaternären Ammoniumverbindungen - zu kombinieren, sind bislang fehlgeschlagen: Es bildeten sich stets unlösliche Addukte. Diese ließen sich zwar durch geeignete Tenside in Wasser dispergieren, die auf diese Weise erhaltenen wässrigen Zubereitungen ("Gebrauchslösungen") waren jedoch trübe und wurden daher vom Kunden nicht akzeptiert. Ferner hinterließen die Gebrauchslösungen auf Oberflächen unlösliche Rückstände, und die Wirksamkeit dieser Zubereitungen war gegenüber Magnesium-monoperphthalat vermindert (und nicht wie erwartet erhöht).

Es war daher Aufgabe der vorliegenden Erfindung, eine verbesserte Wirkstoffkombination für die Verwendung als Desinfektionsmittel bereitzustellen.

Es war überraschend und für den Fachmann nicht vorhersehbar, dass die Verwendung von Wirkstoffkombinationen mit den Merkmalen des Anspruch 1 die Nachteile des Standes der Technik behebt.

Es war insbesondere überraschend, daß die erfindungsgemäßen tert.-Alkylaminoxide mit Magnesiummonoperphthalat keine unlöslichen Addukte bilden. Überraschend war ebenfalls, dass sofort eine Verbesserung der Wirksamkeit gegen Hefepilze - wie z. B. gegen Candida albicans - eintritt und nicht erst nach einiger Zeit (z. B. nach einer Zersetzung des Peroxids).

Erfindungsgemäß bevorzugte tert.-Alkylaminoxide sind N,N-Dimethyllaurylamin-N-oxid, N,N-Dimethylmyristylamin-N-oxid, N,N-Dimethylpalmitylamin-N-oxid, N,N-Dimethylstearylamin-N-oxid, N,N-Dimethyloleylamin-N-oxid, 4-Decylmorpholin-N-Oxid, 4-Dodecylmorpholin-N-oxid, 4-Tetradecylmorpholin-N-oxid, 4-Hexadecylmorpholin-N-oxid, 4-Oleylmorpholin-N-oxid sowie die entsprechenden N,N-Diethylverbindungen. Die Aminoxide können entweder einzeln oder als Gemische verwendet werden. Vorteilhaft im Sinne der vorliegenden Erfindung sind insbesondere auch handelsübliche Gemische verschiedener Kettenlängen.

Das Verhältnis von tert.-Alkylaminoxid zu Peroxid kann im Prinzip beliebig gewählt werden. Es ist aber vorteilhaft im Sinne der vorliegenden Erfindung, wenn der Anteil des Peroxids überwiegt. Vorteilhaft wird das Verhältnis von tert.-Alkylaminoxid zu Peroxid wie 0,5 : 99,5 bis 15 : 85 gewählt.

Zur Herstellung von Desinfektionsmitteln auf der Basis der erfindungsgemäßen Wirkstoffkombinationen können als weitere vorteilhafte Bestandteile vor allem Tenside und Bindemittel verwendet werden. Erfindungsgemäß bevorzugte Tenside sind die Natriumsalze von organischen Sulfonaten und Schwefelsäureestern - wie z. B. Natrium-Alkylsulfat, -Alkylsulfonate - Benzol-, Toluol-, Cumol-, Xylolsulfonsäurederivate, Isethionate, Naphthalinsulfonate, Alkylarylsulfonate, Alkylethersulfate, Dioctylsulfosuccinat Sulfosuccinatmonoester-EO-decanol, -dodecanol, das Dinatriumsalz des ethoxylierten Nonylphenol, Halbester der Sulfobernsteinsäure, Sarcosinate, Taurate oder Taurinate.

Bevorzugt sind auch nichtionische Tenside wie die Ethylenoxid- oder Propylenoxidderivate von aliphatischen oder gemischt aliphatischen/aromatischen Alkoholen oder Carbonsäuren.

Vorteilhafte weitere Hilfsstoffe sind Bindemittel wie Cellulosederivate und dergleichen.

In einer vorteilhaften Ausführungsform besteht das erfindungsgemäß verwendete Desinfektionsmittel zu 60 bis 85 Gew.-% aus Magnesiummonoperphthalat, zu 0,5 bis 20 Gew.-% aus Aminoxid und zu 0 bis 20 Gew.-% aus sonstigen Komponenten, wie z. B. Tensiden oder Bindemitteln, wobei die Prozentangaben jeweils auf das Gesamtgewicht des Desinfektionsmittels bezogen sind.

Vorteilhafte Desinfektionsmittel auf Basis der erfindungsgemäß verwendeten Wirkstoffkombinationen sind wasserfreie Zubereitungen (Konzentrate) in Form von wasserfreiem Granulat, Pellets oder Tabletten, die unmittelbar vor Gebrauch in Wasser aufgelöst und anschließend als wässrige Gebrauchslösung verwendet werden. Die Wirkstoffkonzentration in den wässrigen Gebrauchslösungen liegt erfindungsgemäß zwischen 0,05 und 5 Gew.-%, vorzugsweise zwischen 0,1 und 1,0 Gew.-% der Wirkstoffkombination, bezogen auf das Gesamtgewicht der Gebrauchslösung.

Die wasserfreien Zubereitungen sind bei Raumtemperatur und wasserdichter Verpackung mehrere Jahre haltbar (lagerbar), die wässrigen Gebrauchslösungen behalten ihre Wirksamkeit für etwa 8 bis 24 Stunden.

Unter "wasserfrei" wird im Sinne der vorliegenden Erfindung verstanden, dass den Zubereitungen keinerlei Wasser zugesetzt wird, sondern Wasser lediglich als "Verunreinigung" über die verwendeten Rohstoffe in die Zubereitungen eingeschleppt wird, so daß außer Kristallwasser in den Peroxiden keinerlei Wasser in den Zubereitungen vorhanden sein sollte.

Der große Vorteil der erfindungsgemäßen Verwendung ist das breite Wirkungsspektrum und die hervorragende Verträglichkeit, sowohl gegenüber der menschlichen Haut als auch gegenüber den verschiedenartigsten Materialien. Deswegen sind Desinfektionsmittel auf der Basis der vorliegenden Erfindung sehr breit anwendbar: insbesondere als Hände-, Haut-, Schleimhaut- oder Wunddesinfektionsmittel, als Kontaktlinsendesinfektionsmittel, als Flächendesinfektionsmittel und/oder als Instrumentendesinfektionsmittel.

Die folgenden Beispiele dienen dazu, die Erfindung zu beschreiben, selbstverständlich ohne dass beabsichtigt ist, die Erfindung auf diese Beispiele zu beschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

### Beispiele 1 - 5

Es wurden folgende Wirkstoffkombinationen durch Vermischen wässriger Lösungen herge-stellt:

| **Beispiel** | **Vergleich** | **1** | **2** | **3** | **4** | **5** |
|---|---|---|---|---|---|---|
| MMPP | 100% | 90% | 75% | 50% | 25% | 5% |
| Barlox 12 | - | 10% | 25% | 50% | 75% | 95% |

| | | | | | | |
|---|---|---|---|---|---|---|
| MMPP = Magnesiummonoperphthalat Barlox 12 = N-Dodecyl-N,N-Dimethyl-N-oxid | | | | | | |

Von diesen Mischungen wurden verdünnte Lösungen hergestellt (mit Wirkstoffkonzentrationen von 0,01 Gew.-%, 0,025 Gew.-% und 0,05 Gew.-%, jeweils bezogen auf das Gesamtgewicht der verdünnten Lösung, s.u.) und die mikrobizide Wirksamkeit mit Hilfe des quantitativen Suspensionstes nach den Standardmethoden der DGHM (Deutschen Gesellschaft für Hygiene und Mikrobiologie) zur Prüfung chemischer Desinfektionsverfahren vom 1. September 2001 geprüft. Als Prüfkeime wurden verwendet:
Staphylococcus aureus ATCC6538
Escherichia coli K 12 ATCC 10538
Enterococcus hirae ATCC 10451
Pseudomonas aeroginosa ATCC 15442 und
Candida albicans ATCC 10231

### Ergebnisse

| Staphylococcus aureus, Reduktionsfaktoren, 30 / 60 Minuten Einwirkzeit | | | | | | |
|---|---|---|---|---|---|---|
| Konz./Beisp. | Vergleich | 1 | 2 | 3 | 4 | 5 |
| 0,01% | 0,2 / 0,27 | 0,09 / 0,31 | 0,19 / 0,68 | 0,16 / 0,27 | 0,09 / 0,50 | 0,12 / 0,16 |
| 0,025% | 0,29 / 0,72 | 0,24 / 0,44 | 0,26 / 0,34 | 0,89 / 4,03 | 1,14 / 2,75 | 1.09 / 1,97 |
| 0,05% | 0,61 / 3,05 | 4,39 / 4,81 | 3,24 / 4,81 | 3,6 / 4,54 | 3,21 / 5,11 | 1,91 / 3,43 |

Die gleichen Werte wurden auch für Escherichia coli, Enterococcus hirae und Pseudomonas aeruginosa erhalten.

| Candida albicans, Reduktionsfaktoren, 30 / 60 Minuten Einwirkzeit | | | | | | |
|---|---|---|---|---|---|---|
| Konz./Beisp. | Vergleich | 1 | 2 | 3 | 4 | 5 |
| 0,1% | 0,66 / 0,57 | 3,96 />4,95 | >5,04/>4,95 | >5,04/>4,95 | >5,04/>4,95 | >5,04/>4,95 |
| 0,25% | 0,74 / 0,68 | >5,04/>4,95 | >5,04/>4,95 | >5,04/>4,95 | >5,04/>4,95 | >5,04/>4,95 |
| 0,5% | 1,59 / 4,65 | >5,04/>4,95 | >5,04/>4,95 | >5,04/>4,95 | >5,04/>4,95 | >5,04/>4,95 |
| 0,75% | >5,04/>4,95 | >5,04/>4,95 | >5,04/>4,95 | >5,04/>4,95 | >5,04/>4,95 | >5,04/>4,95 |

### Beispiel 6

Es wurden Granulate mit folgenden Zusammensetzungen hergestellt:

| | Beispiel | Vergleich |
|---|---|---|
| Magnesiummonoperphthalat | 80 | 80 Gew.% |
| Barlox 12 | 1,5 | - Gew.% |
| Marlipal 0 13/90 | 1,5 | 1,6 |
| Marlon ARL | 7,0 | 7,4 |
| Natrium Cumolsulfonat | 8,84 | 9,84 |
| Wallocel MW 6000 GB | 1,16 | 1,16 |

Von diesen Granulaten wurden wässrige Lösungen hergestellt (mit Wirkstoffkonzentrationen von 0,1 Gew.-%, 0,25 Gew.-% und 0,5 Gew.-%, jeweils bezogen auf das Gesamtgewicht der wässrigen Lösung, s.u.) und wie vorstehend die Wirksamkeit gegen Staphylococcus aureus und Candida albicans geprüft.

### Ergebnisse

| Reduktionsfaktoren Staphylococcus aureus, 5 min / 30 min / 60 / min | | |
|---|---|---|
| Konzentration | Beispiel 6 | Vergleich |
| 0;5% | 1,54 / >5,73 / >5,21 | 1,76 / >5,73 / >5,21 |
| 0,1% | >5,63 / >5,73 / >5,21 | >5,63 / >5,73 / >5,21 |
| 0,25% | >5,63 / >5,73 / >5,21 | >5,63 / >5,73 / >5,21 |

| Reduktionsfaktoren Candida albicans, 5 min / 30 min / 60 min | | |
|---|---|---|
| Konzentration/Beispiel | Beispiel 6 | Vergleich |
| | | |
| 0,25% | 0,04 / 1,71 / 4,43 | 0,04 / 1,10 / 1,65 |
| 0,5% | 0,06 / >4,41 / >4,41 | 0,01 / 0,08 / 0,21 |
| | | |

Zur Überprüfung der Stabilität wurden die Granulate 6 Monate bei Raumtemperatur gelagert und sodann der Peroxidgehalt bestimmt.
Ergebnis: Der Peroxidgehalt war konstant geblieben.

## Patentansprüche

1. Verwendung von Wirkstoffkombinationen aus
(a) tert.-Alkylaminoxiden der allgemeinen Formel (R¹)(R²)(R³)N-O,
wobei
R¹ eine Alkylkette mit 8 bis 18 Kohlenstoffatomen ist, die geradkettig oder verzweigt, gesättigt oder ungesättigt sein kann und
R² und R³ entweder Methyl- oder Ethylgruppen sind, und
(b) Peroxiden als Desinfektionsmittel,
**dadurch gekennzeichnet, dass** als Peroxid Magnesiummonoperphthalat gewählt wird.

2. Verwendung nach Anspruch 1, **dadurch gekennzeichnet, dass** das Gewichtsverhältnis von Peroxid zu tert.-Alkylamin-N-Oxid aus dem Bereich von 99,5 zu 0,5 bis 0,5 zu 99,5, vorzugsweise zwischen 99,5 zu 0,5 bis 85 zu 15 gewählt wird.

3. Verwendung nach einem der vorhergehenden Ansprüche, **dadurch gekennzeichnet, dass** das Desinfektionsmittel als wasserfreie Zubereitung in Form von Granulat, Pellets oder Tabletten bereitgestellt und unmittelbar vor Gebrauch in Wasser aufgelöst und anschließend als wässrige Gebrauchslösung verwendet wird.

4. Verwendung nach einem der vorhergehenden Ansprüche, **dadurch gekennzeichnet, dass** R¹ eine geradkettige oder verzweigte, gesättigte oder ungesättigte Alkylkette mit 10 bis 18 Kohlenstoffatomen darstellt.

5. Verwendung nach einem der vorhergehenden Ansprüche, **dadurch gekennzeichnet, dass** das Desinfektionsmittel aus 60 bis 85 Gew. % Magnesiummonoperphthalat, 0,5 bis 20 Gew.% Aminoxid und 0 bis 20 Gew.% sonstigen Komponenten, wie z. B. Tensiden oder Bindemitteln, besteht.

## Claims

1. Use of active ingredient combinations of
(a) tert-alkylamine oxides of the general formula (R¹) (R²) (R³) N-O,
where
R¹ is an alkyl chain having 8 to 18 carbon atoms, which can be straight-chain or branched, saturated or unsaturated, and
R² and R³ are either methyl or ethyl groups, and
(b) peroxides as disinfectants,
**characterized in that** magnesium monoperphthalate is selected as peroxide.

2. Use according to Claim 1, **characterized in that** the weight ratio of peroxide to tert-alkylamine N-oxide is selected from the range of from 99.5:0.5 to 0.5:99.5, preferably between 99.5:0.5 and 85:15.

3. Use according to one of the preceding claims, **characterized in that** the disinfectant is provided as anhydrous preparation in the form of granules, pellets or tablets and is dissolved in water directly prior to use and is then used as aqueous use solution.

4. Use according to one of the preceding claims, **characterized in that** R¹ is a straight-chain or branched, saturated or unsaturated alkyl chain having 10 to 18 carbon atoms.

5. Use according to one of the preceding claims, **characterized in that** the disinfectant consists of 60 to 85% by weight of magnesium monoperphthalate, 0.5 to 20% by weight of amine oxide and 0 to 20% by weight of other components, such as e.g. surfactants or binders.

## Revendications

1. Utilisation d'associations de substances actives composées
(a) d'oxydes de tert-alkylamines de formule générale (R¹) (R²) (R³) N-O,
où
R¹ est une chaîne alkyle ayant de 1 à 18 atomes de carbone, qui peut être à chaîne droite ou ramifiée, saturée ou insaturée et
R² et R³ représentent soit le groupe méthyle soit le groupe éthyle, et
(b) de peroxydes en tant que désinfectant,
**caractérisée en ce qu'**on choisit comme peroxyde le monoperphtalate de magnésium.

2. Utilisation selon la revendication 1, **caractérisée en ce que** le rapport pondéral du peroxyde au N-oxyde de tert-alkylamine est choisi dans la plage de 99,5 : 0,5 à 0,5 : 99,5, de préférence entre 99,5 : 0,5 à 85 : 15.

3. Utilisation selon l'une quelconque des revendications précédentes, **caractérisée en ce que** le désinfectant est préparé en tant que préparation aqueuse sous forme de produit granulé, granules ou comprimés et dissous dans de l'eau immédiatement avant l'emploi et ensuite utilisé sous forme de solution aqueuse prête à l'emploi.

4. Utilisation selon l'une quelconque des revendications précédentes, **caractérisée en ce que** R¹ représente une chaîne alkyle à chaîne droite ou ramifiée ayant de 10 à 18 atomes de carbone.

5. Utilisation selon l'une quelconque des revendications précédentes, **caractérisée en ce que** le désinfectant est constitué de 60 à 85 % en poids de monoperphtalate de magnésium, 0,5 à 20 % en poids d'oxyde d'amine et 0 à 20 % en poids d'autres composants, comme par exemple des tensioactifs ou des liants.
